Europäisches Patentamt

European Patent Office

Office européen des brevets

(19)

(11) Veröffentlichungsnummer: **0 173 195**
**B1**

(12) **EUROPÄISCHE PATENTSCHRIFT**

(45) Veröffentlichungstag der Patentschrift: **16.05.90**

(21) Anmeldenummer: **85110328.3**

(22) Anmeldetag: **19.08.85**

(51) Int. Cl.⁵: **A 61 K 7/06**

(54) Haarbehandlungsmittel.

(30) Priorität: **27.08.84 DE 3431417**

(43) Veröffentlichungstag der Anmeldung:
**05.03.86 Patentblatt 86/10**

(45) Bekanntmachung des Hinweises auf die
Patenterteilung:
**16.05.90 Patentblatt 90/20**

(84) Benannte Vertragsstaaten:
**AT BE CH DE FR GB IT LI NL**

(56) Entgegenhaltungen:
EP-A-0 024 799
DE-A-2 109 081
DE-A-2 623 691
US-A-3 400 198
US-A-4 205 063

H. Janistyn, "Handbuch der Kosmetika und
Riechstoffe", Dr. Alfred Hüthig Verlag
Heidelberg, 2. Auflage, III. Band (1973)

(73) Patentinhaber: Henkel Kommanditgesellschaft
auf Aktien
Postfach 1100 Henkelstrasse 67
D-4000 Düsseldorf-Holthausen (DE)

(72) Erfinder: Höffkes, Horst, Dr.
Carlo-Schmid-Strasse 113
D-4000 Düsseldorf (DE)
Erfinder: Giede, Karl
Schlehenweg 12
D-4010 Hilden (DE)

Courier Press, Leamington Spa, England.

**Beschreibung**

Gegenstand der Erfindung sind kosmetische Haarbehandlungsmittel mit einem Zusatz, der die Naßkämmbarkeit des Haares verbessert, die Haarschädigung reduziert und das Haar festigt.

Das Haupthaar weist nach dem Waschen mit Shampoos auf Basis von synthetischen oberflächenaktiven Stoffen oft einen kosmetisch unbefriedigenden Zustand auf: Es fühlt sich stumpf an und ist im nassen Zustand nur schwer zu kämmen. Insbesondere, wenn das Haar durch Färben, Bleichen oder Kaltwellbehandlungen strapaziert ist, wird nach dem Shamponieren eine schlechte Kämmbarkeit und oft eine Verringerung der Festigkeit des Haares beobachtet.

Es ist bekannt, nach dem Waschen, Färben oder Dauerwellen des Haares konditionierende Präparate, sogenannte Haarnachbehandlungsmittel auf das Haar einwirken zu lassen. Als Wirkstoffe sind in solchen Präparaten üblicherweise kationische grenzflächenaktive Stoffe enthalten. Es ist auch bekannt, üblichen Shampoos auf Basis anionaktiver Tenside oder auf Basis von Mischungen von Tensiden unterschiedlicher Jonogenität bestimmte Substanzen beizufügen, um gleichzeitig mit der Haarwäsche einen gewissen konditionierenden Effekt zu erzielen. Unter den Wirkstoffen, die sowohl in Shampoos als auch in Haarnachbehandlungsmitteln zur Verbesserung der kosmetischen Eigenschaften des Haares eingesetzt werden, spielen die wasserlöslichen polykationischen Polymeren eine besondere Rolle. Bekannte polykationische Polymere sind z.B. die aus DE—OS—21 09 081 bekannten synthetischen Polymeren, die in der US—PS—4.292.212 beschriebenen kationischen Guarderivate und die aus US—PS—3.472.840 bekannten, quartären Stickstoff enthaltenden Cellulosether. Die mit diesen Zusätzen erzielbaren Effekte sind jedoch, insbesondere bei stark strapaziertem Haar nicht befriedigend. Darüber hinaus bestand die Aufgabe, Haarbehandlungsmittel zu finden, die der Haarschädigung entgegenwirken und das Haar festigen.

Es wurde gefunden, daß Haarbehandlungsmittel eine erheblich verbesserte kaarkosmetische Wirksamkeit aufweisen, wenn sie neben üblichen Komponenten solcher Mittel eine Kombination aus 0,1 bis 5,0 Gewichtsprozent eines wasserlöslichen kationischen Polymeren und 0,1 bis 10,0 Gewichtsprozent eines wasserlöslichen Aluminiumsalzes enthalten.

Geeignete wasserlösliche kationische Polymere sind z.B. polyquartäre Derivate von Celluloseethern, z.B. gemäß US—PS—3.472.840, Mischpolymerisate von N-Vinylpyrrolidon mit Monomeren, die Dialkylaminoalkylgruppen tragen, z.B. gemäß DE—OS—21 03 899, Polymere und Copolymere des Dialkyldimethylammoniumchlorids, z.B. gemäß DE—OS—21 09 081, Polymere Aminoamide wie sie z.B. durch Kondensation von Adipinsäure, Diethylentriamin und Dimethyl-epoxypropylamin erhalten werden. Polymere der genannten Art sind im Handel z.B. unter der Warenbezeichnung Polymer® IR 400, Gafquat® 755, Merquat® 550, Cartaretin® F4. Weitere geeignete, im Handel erhältliche, wasserlösliche kationische Polymere sind z.B. die Polyglycol-Polyamin-Kondensationsharze, die unter der Warenbezeichnung Polyquart® H 81 erhältlich sind, die kationischen Guarderivate, die unter der Warenbezeichnung Cosmedia® Guar C 261 vertrieben werden, die quartären Cellulosederivate, die unter der Bezeichnung Celquat® L 200 erhältlich sind und die kationischen Polyelektrolyte auf Methacrylat-Basis, die unter der Bezeichnung Rohafloc® KF 720 angeboten werden. Es sind darüber hinaus zahlreiche wasserlösliche kationische Polymere beschrieben worden, die in haarkosmetischen Mitteln avivierende und konditionierende Wirkungen haben. Diese Produkte bewirken in erfindungsgemäßen Haarbehandlungsmitteln in Kombination mit dem wasserlöslichen Aluminiumsalz eine synergistisch gesteigerte Verbesserung der Naßkämmbarkeit, eine Verringerung der Haarschädigung und eine Festigung des Haares.

Die Effekte übertreffen bei weitem die Wirkung, die sich mit den kationischen Polymeren allein oder mit den Aluminiumsalzen allein erzielen lässt, der im Hinblick auf die Verbesserung der Naßkämmbarkeit erreichte Effekt ist z.B. auch stärker als die Summe der mit den beiden Komponenten erzielbaren Einzeleffekte, so daß von einer synergistischen Steigerung der haarkosmetischen Wirkung gesprochen werden kann.

Geeignete wasserlösliche Aluminiumsalze sind z.B. Aluminiumsulfat $[Al_2(SO_4)_3 \cdot 18H_2O]$, Aluminiumchlorid $[AlCl_3 \cdot 6H_2O]$, basische Aluminiumsalze wie z.B. Aluminiumhydroxychlorid $[Al(OH)_2Cl]$, gemischte Aluminiumsalze wie die Alaune, z.B. Kaliumaluminiumsulfat $[KAl(SO_4)_2 \cdot 12H_2O]$, organische Aluminiumsalze, z.B. Aluminiumacetat (essigsaure Tonerde), Aluminiumglycolat Aluminiumlactat, Aluminiumgluconat und komplexe Aluminiumsalze wie z.B. Natriumaluminium-chlorhydroxylactat (Chloracel®).

Aluminiumsalze von Hydroxycarbonsäuren wie z.B. Aluminiumlactat oder Aluminiumgluconat haben den Vorteil, daß die damit hergestellten, erfindungsgemäßen Haarbehandlungsmittel auch im neutralen bis schwach basischen pH-Bereich klar bleiben. Es hat sich jedoch gezeigt, daß eine leichte Austrübung aufgrund der hydrolytischen Spaltung der Aluminiumsalze unter Bildung schwerer löslicher basischer Salze oder Oxidhydrate für die haarkosmetische Wirkung der erfindungsgemäßen Haarbehandlungsmittel nicht nachteilig ist, so lange wenigstens 0,1 Gewichtsprozent Aluminiumsalz in der Lösung verbleibt.

Neben der Kombination aus wasserlöslichen, kationischen Polymeren und wasserlöslichen Aluminiumsalz enthalten die erfindungsgemäßen Haarbehandlungsmittel die in solchen Zubereitungen üblichen Komponenten. Die erfindungsgemäßen Haarbehandlungsmittel werden als Haarwaschmittel (bzw. Shampoos) oder als Haarnachbehandlungsmittel zubereitet. Haarnachbehandlungsmittel im Sinne der Erfindung sind Haarspülungen, Haarkurpräparate und auch Präparate, die nach der Einwirkung auf dem Haar verbleiben, um diesem Halt und Fülle zu verleihen und das Frisieren zu erleichtern, z.B. Haarfestiger und sogenannte Fönwellmittel (Fönlotionen).

EP 0 173 195 B1

Erfindungsgemäße Haarbehandlungsmittel in Form eines Haarwaschmittels enthalten z.B. die üblichen oberflächenaktiven Stoffe, bevorzugt schaumstarke anionische Tenside, jedoch keine Seifen. Bevorzugt werden Aniontenside aus der Gruppe der Alkali-, Ammonium-, Magnesium-, Mono-, Di- oder Triethanolammoniumsalze von Aniontensiden mit einer linearen Alkylgruppe mit 10 bis 18 C-Atomen und einer Sulfat- oder Sulfonatgruppe im Molekül verwendet. Beispiele für Aniontenside, die diese kennzeichnenden Strukturelemente enthalten sind Alkylsulfate mit 10 bis 16 C-Atomen in der Alkylgruppe, Alkylethersulfate mit 12 bis 16 C-Atomen in der Alkylgruppe und 1 bis 10 Glycolethergruppen, Alkansulfonate mit 10 bis 18 C-Atomen, Alken- und Hydroxyalkansulfonate wie sie bei der Sulfonierung von α-Olefinen mit 10 bis 18 C-Atomen erhalten werden, Fettsäurealkylolamid- und Fettsäurealkylolamidpolyglycolether-sulfate Fettsäuremonoglyceridsulfate, Sulfobernsteinsäuremonoalkylester, Acyltauride und Acylisethionate mit 10 bis 18 C-Atomen in der Acylgruppe.

Bevorzugt sind in erfindungsgemäßen Haarbehandlungsmitteln, die Haarwaschmittel darstellen, 5 bis 25 Gewichtsprozent solcher Aniontenside, 0,1 bis 5 Gewichtsprozent eines wasserlöslichen kationischen Polymeren und 0,1 bis 10 Gewichtsprozent eines wasserlöslichen Aluminiumsalzes enthalten.

Daneben können auch Tenside anderer Jonogenität enthalten sein. Besonders bevorzugt ist die Verwendung einer Tensidkombination aus einem Aniontensid und einem ampholytischen oder zwitterionischen Tensid. Ampholytische Tenside sind z.B. N-$C_8$—$C_{18}$-Alkyl-β-aminopropionsäuren, N-$C_8$—$C_{18}$-Alkyl-β-iminodipropionsäuren oder N-Hydroxyethyl-N-kokosacylamidopropylglycin geeignet. Als zwitterionische Tenside (Betaintenside) können z.B. N - Kokosalkyl - dimethyl - glycin oder N - Kokosacylamido - propyldimethyl - glycin eingesetzt werden.

Bevorzugt enthalten erfindungsgemäße Haarbehandlungsmittel, die ein Haarwaschmittel darstellen, eine Tensidkombination aus

10 bis 20 Gewichtsprozent eines Alkylsulfats oder Alkylethersulfats der allgemeinen Formel R—(OC₂H₄)ₙ—OSO₃M, in der R eine lineare Alkylgruppe mit 10 bis 16 C-Atomen, n=0 oder eine ganze Zahl von 1 bis 10 und M ein Natrium, Ammonium oder Triethanolammonium ist, und

1 bis 5 Gewichtsprozent eines ampholytischen oder zwitterionischen Tensids, jeweils bezogen auf das gesamte Haarwaschmittel.

Ein Gehalt von 0,5 bis 5 Gewichtsprozent eines wasserlöslichen Aluminiumsalzes ist in der Regel ausreichend. Daneben enthalten die erfindungsgemäßen Shampoos übliche Zusatz- und Stellmittel wie z.B. Verdickungsmittel von Typ der Fettsäurealkylolamide oder Polyvinylpyrrolidon, Trübungsmittel wie z.B. Ethylenglycoldistearat, pH-Wert-Stabilisatoren (Puffer) wie z.B. Alkali- oder Ammoniumphosphate oder Citrate, Konservierungsmittel wie z.B. Formaldehyd, p-Hydroxybenzoesäureester, Farbstoffe, Duftstoffe sowie übliche haarkosmetische Wirkstoffe wie z.B. Antischuppenwirkstoffe, Sebostatika, Vitamine, Pflanzenextrakte u.s.w.

Haarbehandlungsmittel in Form von Haarspülmitteln sind eine weitere bevorzugte Ausführungsform der Erfindung. Konditionierende Haarnachspülmittel enthalten üblicherweise (monomere) quartäre Ammoniumtenside als konditionierende Wirkstoffe und werden zubereitet als Öl-in-Wasser-Emulsionen von Fettkomponenten (z.B. Cetylalkohol und/oder Stearylalkohol) in Wasser unter Zuhilfenahme nichtionogener Emulgatoren und Schutzkolloide. Erfindungsgemäße Haarnachspülmittel können analog formuliert werden, wobei als konditionierender Wirkstoff die Kombination aus kationischem Polymeren und Aluminiumsalz eingesetzt wird. Bevorzugt werden erfindungsgemäße Haarnachspülmittel jedoch nicht als Emulsion, sondern als wäßrige Lösung formuliert, die Aniontenside, ampholytische und/oder zwitterionische Tenside und die Kombination aus polykationischem Polymeren und wasserlöslichem Aluminiumsalz enthält. Besonders wirksam sind erfindungsgemäße Haarbehandlungsmittel, die ein Haarnachspülmittel darstellen, mit einem Gehalt von

1 bis 10 Gewichtsprozent eines anionischen Tensids in Form eines Alkali-, Ammonium-, Magnesium-, Mono-, Di- oder Triethanolammoniumsalzes eines Aniontensids mit einer linearen Alkylgruppe mit 10 bis 18 C-Atomen und einer Sulfat- oder Sulfonatgruppe im Molekül

0 bis 5 Gewichtsprozent eines ampholytischen oder zwitterionischen Tensids

0,1 bis 5 Gewichtsprozent eines polykationischen Polymeren und

0,1 bis 10 Gewichtsprozent eines wasserlöslichen Aluminiumsalzes.

Beispiele für in Frage kommende Aniontenside, ampholytische und zwitterionische Tenside wurden bereits vorher genannt. Das Mengenverhältnis von Aniontensiden zu ampholytischen und/oder zwitterionischen Tensiden kann 4:1 bis 1:4 betragen, die Gesamtmenge an Tensiden kann in solchen Haarnachspülmitteln 1 bis 10 Gewichtsprozent ausmachen.

Neben den kennzeichnenden Bestandteilen können die erfindungsgemäßen Haarnachspülmittel übliche Hilfsmittel wie z.B. Verdickungsmittel, Trübungsmittel, Farbstoffe, Duftstoffe, Konservierungsmittel und haarkosmetische Wirkstoffe wie z.B. Antischuppenwirkstoffe, Sebostatika, Vitamine, Pflanzenextrakte u.s.w. enthalten.

Erfindungsgemäße Haarnachbehandlungsmittel, die nach der Anwendung auf dem Haar verbleiben, wie z.B. Haarfestiger und Fönwellmittel, enthalten bevorzugt niedere Alkohole, insbesondere Ethanol und/oder Isopropanol in den in solchen Mitteln üblichen Mengen, dies sind für Festiger und Fönwellmittel 10 bis 50 Gewichtsprozent. Festiger und Fönwellmittel enthalten zusätzlich haarfestigende anionische und/oder nichtionogene filmbildende Polymerer, z.B. Polyvinylpyrrolidone (z.B. Luviskol®) oder Copolymere aus

3

Maleinsäure und Acryl- oder Methacrylsäureestern, in Mengen von 0,1 bis 1,0 Gewichtsprozent, bezogen auf das gesamte Mittel. Bei Aerosolpräparaten beziehen sich die genannten Prozentsätze auf die treibmittelfreie Zubereitung.

Die folgenden Beispiele sollen den Gegenstand der Erfindung näher erläutern ohne ihn hierauf zu beschränken.

Beispiele
1. Anwendungsbeispiele
1.1 Haarshampoo für strapaziertes Haar

| Zusammensetzung: | Gew.-% |
|---|---|
| Fettalkohol ($C_{12}$—$C_{14}$)+2 EO-sulfat, Na-Salz (28 %ig) | 64,0 |
| Kokosacylamidopropyl-dimethylglycin (30 %ig) | 5,0 |
| Lanolin-Oxethylat (Solan® E) | 1,0 |
| Polyquartäres Cellulosederivat (Polymer JR 400®) | 0,5 |
| Aluminium-Salz | 2,0 |
| Duftstoff | 0,5 |
| Wasser | 27,0 |

1.2 Haarnachspülmittel

| Zusammensetzung | Gew.-% |
|---|---|
| Fettalkohol ($C_{12}$—$C_{14}$)+2 EO-sulfat, Na-Salz (28 %ig) | 10,0 |
| Kokosacylamidopropyl-dimethylglycin (30 %ig) | 5,0 |
| Polyquartäres Cellulosederivat (Polymer JR 400®) | 0,25 |
| Aluminium-Salz | 0,25 |
| Wasser | 84,5 |

In die Rezepturen 1.1 und 1.2 wurden als Aluminiumsalz sowohol Aluminium-Lactat als auch $Al_2(SO_4)_3 \cdot 18H_2O$ eingesetzt.

1.3 Fönwellmittel

| Zusammensetzung | Gew.-% |
|---|---|
| Isopropylalkohol | 10,0 |
| Gafquat® 755 | 1,5 |
| Aluminiumhydroxychlorid | 0,5 |
| Polyvinylpyrrolidon (Luviskol® K30) | 0,3 |
| Duftstoffe | 0,5 |
| Wasser | 87,2 |

2. Prüfung der Verringerung der Haarschädigung

In Versuchsreihe 1 wurden Haarsträhnen des Typs Natural Light European (Code 6633, Fa. Alkinco) je 3×dauergewellt und 18×shampooniert und anschliessend die auf das Shampoonieren zurückzuführenden Gewichtsverluste bestimmt—jeweils bezogen auf das Trockengewicht des dauergewellten Haares—getrennt nach Wurzel- und Spitzenbereich.

Als Shampoobasis (Vergleichsshampoo) wurde das Produkt nach Beispiel 1.1 ohne den Zusatz von Aluminium-Salz eingesetzt. Das Prüfshampoo enthielt 2% Al-Lactat.
Folgende Ergebnisse wurden erhalten:

| Gewichtsdifferenzen (%), bezogen auf das Trockengewicht | | | |
|---|---|---|---|
| Vergleich (ohne Al-Salz) | | Vergleich+2% Al-Lactat | |
| Wurzelbereich | Spitzenbereich | Wurzelbereich | Spitzenbereich |
| −0,11±0,03 | −0,25±0,11 | +0,66±0,19 | +0,76±0,17 |

Bei dem Al-haltigen Shampoo erfolgt im Gegensatz zum Vergleichsshampoo keine Gewichtsabnahme, sondern eine Gewichtszunahme, die wahrscheinlich auf die Einlagerung von Al-Verbindungen ins Haar zurückzuführen ist. (Al-Gehalt 0,12% bzw. 0,15% für den Wurzel- bzw. den Spitzenbereich).

In Versuchsreiche 2 wurden Last-Dehnungsdiagramme von 5×kaltgewellten und 25×shampoonierten Haaren aufgenommen. Jeweils 15 Haare wurden vermessen.

Die folgenden Unterschiede zum Vergleich wurden ermittelt:

| | | | Biegemodul | |
|---|---|---|---|---|
| | Reißkraft/ Fläche | Gesamtdehnung | kleine Achse | große Achse |
| Vergleich + 2% Al-Lactat | +0,8% | +20% | +10% | +20% |
| Vergleich + 2% Al-Gluconat | +18,1% | +11,5% | +15% | +17% |

3. Prüfung der Verbesserung der Naßkämmbarkeit

Shampoos mit folgender Basiszusammensetzung wurden auf ihre naßkämmbarkeitsverbessernden Eigenschaften untersucht:

| | 1 | 2 | 3 | 4 |
|---|---|---|---|---|
| Texapon NSW | 64% | 64% | 64% | 64% |
| Dehyton K | 5% | 5% | 5% | 5% |
| Polymer IR 400® | — | — | 0,5% | 0,5% |
| $Al_2(SO_4)_3 \cdot 18H_2O$ | — | 2,0% | — | 2,0% |
| Wasser, Parfümöl | ad 100% | ad 100% | ad 100% | ad 100% |

Die folgenden relativen Kämmkräfte, bezogen auf den Ausgangszustand, wurden analog der in J. Soc. Cosmet. Chem. *27* (1976), Seite 379—398 angegebenen Methode gemessen:

| Shampoo Nr./Zusatz | relative Kämmbarkeit |
|---|---|
| 1 (O-Shampoo) | 106% |
| 2 (+$Al_2(SO_4)_3$) | 98% |
| 3 (+Polymer IR) | 88% |
| 4 (+$Al_2(SO_4)_3$+Polymer IR) | 70% |

**Patentansprüche**

1. Haarbehandlungsmittel, dadurch gekennzeichnet, daß neben üblichen Komponenten solcher Mittel eine Kombination aus

0,1 bis 5,0 Gewichtsprozent eines wasserlöslichen kationischen Polymeren und

0,1 bis 10 Gewichtsprozent eines wasserlöslichen Aluminiumsalzes enthalten ist.

2. Haarbehandlungsmittel nach Anspruch 1, dadurch gekennzeichnet, daß es ein Haarwaschmittel mit einem Gehalt von

5 bis 25 Gewichtsprozent eines anionischen Tensids in Form eines Alkali-, Ammonium-, Magnesium, Mono-, Di- oder Triethanolammoniumsalzes eines Tensidanions mit einer linearen Alkylgruppe mit 10 bis 18 C-Atomen und einer Sulfat- oder Sulfonatgruppe im Molekül

0,1 bis 5 Gewichtsprozent eines wasserlöslichen kationischen Polymeren und

0,1 bis 10 Gewichtsprozent eines wasserlöslichen Aluminiumsalzes darstellt.

3. Haarbehandlungsmittel nach Anspruch 1 oder 2, dadurch gekennzeichnet, daß es ein Haarwaschmittel mit einem Gehalt von

10 bis 20 Gewichtsprozent eines Alkylsulfats oder Alkylethersulfats der allgemeinen Formel $R—(OC_2H_4)_n—OSO_3M$, in der R eine lineare Alkylgruppe mit 10 bis 16 C-Atomen, n=0 oder eine ganze Zahl von 1 bis 10 und M ein Natrium, Ammonium oder Triethanolammonium ist,

1 bis 5 Gewichtsprozent eines ampholytischen oder zwitterionischen Tensids

darstellt.

4. Haarbehandlungsmittel nach Anspruch 1, dadurch gekennzeichnet, daß es ein Haarnachspülmittel mit einem Gehalt von

1 bis 10 Gewichtsprozent eines anionischen Tensids in Form eines Alkali-, Ammonium-, Magnesium-, Mono-, Di- oder Triethanolammoniumsalzes eines Tensidanions mit einer linearen Alkylgruppe mit 10 bis 18 C-Atomen und einer Sulfat- oder Sulfonatgruppe im Molekül

0 bis 5 Gewichtsprozent eines ampholytischen oder zwitterionischen Tensids

0,1 bis 5 Gewichtsprozent eines wasserlöslichen kationischen Polymeren und

0,1 bis 10 Gewichtsprozent eines wasserlöslichen Aluminiumsalzes

darstellt.


**Revendications**

1. Composition pour le traitement des cheveux, caractérisée en ce qu'elle renferme, en plus des composants usuels de telles compositions, une combinaison de:

0,1 à 5,0% en poids d'un polymère cationique soluble dans l'eau et,

0,1 à 10% en poids d'un sel d'aluminium soluble dans l'eau,

2. Composition pour le traitement des cheveux selon la revendication 1, caractérisée en ce qu'elle correspond à un produit de lavage des cheveux renfermant:

5 à 25% en poids d'un agent tensio-actif anionique sous la forme d'un sel alcalin, d'ammonium, de magnésium, de mono-, de di- ou de triéthanolammonium d'un anion tensio-actif ayant dans sa molécule un radical alcoyle linéaire avec 10 à 18 atomes de carbone et un groupe sulfate ou sulfonate,

0,1 à 5% en poids d'un polymère cationique soluble dans l'eau et,

0,1 à 10% en poids d'un sel d'aluminium soluble dans l'eau.

3. Composition de traitement des cheveux selon la revendication 1 ou 2, caractérisée en ce qu'elle correspond à un produit de lavage des cheveux renfermant:

10 à 20% en poids d'un alcoylsulfate ou d'un alcoyléthersulfate de formule générale: $R(OC_2H_4)_n—O—SO_3M$ dans laquelle R est un radical alcoyle linéaire ayant de 10 à 16 atomes de carbone, n est égal à 0 ou est un nombre entier de 1 à 10, et M est ion sodium, ammonium ou triéthanolammonium,

1 à 5% en poids d'un agent tensio-actif amphotère ou hermaphrodite.

4. Composition de traitement des chevaux selon la revendication 1, caractérisée en ce qu'elle correspond à un produit de rinçage final des cheveux renfermant:

1 à 10% en poids d'un agent tensio-actif anionique sous la forme d'un sel alcalin, d'ammonium, de magnésium, de mono-, de di- ou de triéthanolammonium d'un anion tensio-actif ayant dans sa molécule un radical alcoyle linéaire comportant 10 à 18 atomes de carbone et un groupe sulfate ou sulfonate dans la molécule,

0 à 5% en poids d'un agent tensio-actif amphotère ou hermaphrodite,

0,1 à 5% en poids d'un polymère cationique soluble dans l'eau,

0,1 à 10% en poids d'un sel d'aluminium soluble dans l'eau.

**Claims**

1. A hair treatment preparation, characterized in that, in addition to components typical of such preparations, it contains a combination of
0.1 to 5.0% by weight of a water-soluble cationic polymer and
0.1 to 10% by weight of a water-soluble aluminium salt.

2. A hair treatment preparation as claimed in claim 1, characterized in that it is in the form of a shampoo containing
5 to 25% by weight of an anionic surfactant in the form of an alkali, ammonium, magnesium, mono-, di- or triethanolammonium salt of a surfactant anion containing a linear $C_{10-18}$ alkyl group and a sulfate or sulfonate group in the molecule,
0.1 to 5% by weight of a water-soluble cationic polymer and
0.1 to 10% by weight of water-soluble aluminium salt.

3. A hair treatment preparation as claimed in claim 1 or 2, characterized in that it is in the form of a shampoo containing
10 to 20% by weight of an alkyl sulfate or alkyl ether sulfate having the general formula $R-(OC_2H_4)_n-OSO_3M$, in which R is a linear $C_{10-16}$ alkyl group, n=0 or an integer of 1 to 10 and M is a sodium, ammonium or triethanolammonium ion,
1 to 5% by weight of an ampholytic or zwitterionic surfactant.

4. A hair treatment preparation as claimed in claim 1, characterized in that it is in the form of a hair rinse containing
1 to 10% by weight of an anionic surfactant in the form of an alkali, ammonium, magnesium, mono-, di- or triethanolammonium salt of a surfactant anion containing a linear $C_{10-18}$ alkyl group and a sulfate or sulfonate group in the molecule
0 to 5% by weight of an ampholytic or zwitterionic surfactant
0.1 to 5% by weight of a water-soluble cationic polymer and
0.1 to 10% by weight of a water-soluble aluminium salt.